# EUROPÄISCHE PATENTSCHRIFT

(11) **EP 3 930 770 B1**
(45) Veröffentlichungstag und Bekanntmachung des Hinweises auf die Patenterteilung: **06.11.2024**
(21) Anmeldenummer: 20703434.9
(22) Anmeldetag: 03.02.2020
(51) Int. Cl.: A61L 15/18, A61L 15/42, A61L 15/54

(54) **PASTE FÜR DIE MARKIERUNG VON RÖNTGENKONTRASTUNFÄHIGEN WUNDAUFLAGEN**
PASTE FOR MARKING WOUND DRESSINGS INCAPABLE OF X-RAY CONTRAST
PÂTE POUR LE MARQUAGE DE PANSEMENTS SANS POUVOIR DE CONTRASTE

(30) Priorität: 28.02.2019 DE 102019105111
(43) Veröffentlichungstag der Anmeldung: 05.01.2022
(73) Patentinhaber: Speed Care Mineral GmbH, 17034 Neubrandenburg (DE)
(72) Erfinder: SCHOMBURG, Joachim, 17034 Neubrandenburg (DE); SCHULTZ, Christian, 17033 Neubrandenburg (DE); FROHN, Wolfgang, 95197 Schauenstein (DE)
(74) Vertreter: Hertin und Partner Rechts- und Patentanwälte PartG mbB
(86) Internationale Anmeldenummer: PCT/EP2020/052573
(87) Internationale Veröffentlichungsnummer: WO 2020/173665

(56) Entgegenhaltungen:
- WO-A1-2019/016367
- US-A1- 2013 344 131

## Beschreibung

### Technisches Gebiet

Die Erfindung betrifft eine Paste für die Markierung von textilen Gebilden und/oder anderweitigen röntgenkontrastunfähigen Erzeugnissen. Die Paste umfasst dabei die Mineralstoffe Baryt (BaSO₄) und Halloysit. Des Weiteren betrifft die Erfindung eine Wundauflage und ihre Verwendung zur Versorgung von Wundverletzungen. Die Wundauflage beinhaltet Verbundfasern und ist mit der eingangs beschriebenen Paste versehen. Außerdem betrifft die Erfindung ein Verfahren zur Aufbringung der eingangs beschriebenen Paste auf einem textilen Gebilde und/oder anderweitigen Erzeugnissen.

### Hintergrund und Stand der Technik

Im Stand der Technik sind eine Vielzahl an Wundauflagen für Wundverletzungen bekannt. Viele Mittel für die Behandlung von Wundverletzungen umfassen beispielsweise Bandagen aus Baumwolle oder anderen Materialien. Des Weiteren gibt es sogenannte Mullbinden, die eine Träger-Gaze aufweisen, die Chitosan-beschichtet ist. Weiterhin sind blutstillende Verbände bekannt, die mit Kaolin beschichtet sind, die die Blutgerinnung beschleunigen. Neben Kaolin können zur Beschichtung von Verbänden auch Zeolithgranulate, Dreischichtsilikate oder Diatomeenerde verwendet werden. Eine weitere mineralbasierte Wundauflage ist aus der WO 2019/016367 A1 bekannt. Die dort offenbarte Wundauflage umfasst Verbundfasern, die mit Halloysiten beschichtet sind, wobei die Wundauflage gute Eigenschaften zur blutstillenden Wirkung bei der Wundversorgung aufweist.

Nachteilig können derartigen Wundauflagen bei Röntgenuntersuchungen in der Medizin jedoch nicht detektiert werden. Die Röntgendiagnostik ist ein weit verbreitetes bildgebendes Verfahren, bei dem ein Körper oder Teile eines Körpers unter Verwendung eines Röntgenstrahles durchstrahlt wird. Die Durchdringung des Körpers mit Röntgenstrahlen wird in Bildern dargestellt, die als Röntgenbilder bezeichnet werden. In der Medizin dient das Röntgen zur Feststellung von Anomalien im Körper, die im Zusammenhang mit Symptomen, Zeichen und eventuell anderen Untersuchungen eine Diagnose ermöglichen (Röntgendiagnostik). Die unterschiedlich dichten Gewebe des menschlichen (oder tierischen) Körpers absorbieren die Röntgenstrahlen unterschiedlich stark, so dass man eine Abbildung des Körperinneren erreicht. Für die Erkennung von Wundauflagen auf einem Röntgenbild bei beispielsweise inkorporalen Applikationen, müssen die Wundauflagen ebenfalls röntgenkontrastfähige Eigenschaften aufweisen.

Die Absorption von Röntgenstrahlung ist abhängig von ihrem Energieniveau und steigt mit der Anzahl der "im Weg liegenden" Atome, also der Dicke des Objekts und seiner Atom-Dichte sowie der Ordnungszahl Z und Massenzahl A der Atome des Materials. Kontrastmittel weisen eine hohe Absorption der Röntgenstrahlen auf und werden dadurch im Röntgenbild sichtbar. Bariumsulfat (BaSO₄) und Jodverbindungen sind insbesondere in der Medizin häufig eingesetzte Kontrastmittel.

Röntgenkontrastfähige Wundauflagen sind aus der US 4718897 A1, US 4185626 A, EP 2567683 A1, EP 21 47046 B1, DE 19545289 A1 und US 4639253 A bekannt. In den erwähnten Dokumenten werden BaSO₄ aufweisende Thermoplaste verwendet, die als Fasern, Fäden oder Garne extrudiert werden und mit einer Temperatur von 100°C bis 150°C erhitzt, sodass diese polymerisiert werden und auf Kunststoffe oder Textilien aufgebracht werden.

In den Druckschriften DE 19857149 A1, DE 19940862 A1 und EP 1141129 A1 werden ebenfalls Thermoplaste beschrieben, die auf Textilien angebracht werden. Die offenbarten Thermoplaste weisen Jodverbindungen als Kontrastmittel auf.

Die EP 0 272 901 B1 offenbart eine röntgenkontrastfähige Suspension, die über Druckverfahren auf eine Wundauflage aufgebracht wird. Die Suspension enthält einen großen Anteil an Thermoplasten, sodass diese anschließend in einem Heißprozess polymerisiert werden.

Nachteilig an den im Stand der Technik beschriebenen röntgenkontrastfähigen Ausführungen ist der hohe Energiebedarf durch die Erhitzung des Materials zur Polymerisation der Thermoplaste.

Ferner weisen Thermoplaste im Hinblick auf die Umwelt besonders negative Aspekte auf. Die Herstellung von Thermoplasten erfordert einen hohen Bedarf an nichterneuerbaren Ressourcen wie Erdöl oder Erdgas. Zudem sind Thermoplaste biologisch nicht abbaubar, sodass sie bei Eindringen in die Umwelt einen Schaden verursachen. Thermoplaste können nur zersetzt und zerkleinert werden, wodurch sie als Mikroplastik fortbestehen und so auch in die Nahrungskette gelangen.

Ein weiterer Nachteil des Einsatzes von Thermoplasten kann dadurch entstehen, dass dieser unter Umständen zu einer Körperunverträglichkeit führen kann. Bei der inkorporalen Anwendung einer Wundauflage können die Thermoplaste gegebenenfalls Entzündungen hervorrufen oder aber auch krebserregend sein.

Über die Medizin hinaus existieren weitere Anwendungsfelder von Röntgenstrahlen. So werden beispielweise Röntgenstrahlen in der Lebensmittelindustrie eingesetzt, um verpackte Produkte auf nicht beabsichtigten Inhalt zu kontrollieren. Ferner werden in der Flugindustrie Gepäckkontrollen anhand von Röntgenstrahlen durchgeführt. In keinem dieser Anwendungsfelder sind Mittel bekannt, um röntgenkontrastunfähige Materialien zu kennzeichnen, sodass diese in einem Röntgenbild ersichtlich sind. So weisen diese Industrien ebenfalls einen Bedarf an einer Markierung, die röntgenkontrastunfähige Gegenstände unter Röntgenstrahlen kennzeichnet, auf. Beispielweise kann in der Flugindustrie ein schon vor dem Einsatz von Röntgenstrahlen durch Sicherheitsleute begutachteter und für ungefährlich erachteter Gegenstand markiert werden. Anschließend könnte er im Röntgenbild, in dem dieser Gegenstand ohne Markierung als gefährlich eingestuft werden würde, als schon kontrolliert und ungefährlich erachtet werden.

### Aufgabe der Erfindung

Aufgabe der Erfindung ist es somit die Nachteile des Standes der Technik zu beseitigen und ein umweltschonendes sowie energiebedarfarmes Mittel bereitzustellen, welches die Detektierbarkeit von textilen Gebilden und/oder anderweitigen röntgenkontrastunfähigen Erzeugnissen im Röntgenbild gewährleistet.

### Zusammenfassung der Erfindung

Die erfindungsgemäße Aufgabe wird gelöst durch die Merkmale der unabhängigen Ansprüche. Vorteilhafte Ausgestaltungen der Erfindung sind in den abhängigen Ansprüchen beschrieben.

Die Erfindung betrifft in einem ersten Aspekt eine Paste für die Markierung von textilen Gebilden und/oder anderweitigen röntgenkontrastunfähigen Erzeugnissen dadurch gekennzeichnet, dass die Paste Mineralstoffe umfassend Baryt (BaSO₄) und Halloysit in einem Verhältnis von vier Volumenteilen Baryt zu einem Volumenteil Halloysit, aufweist.

Die Paste ist besonders vorteilhaft röntgenkontrastfähig sowie umweltschonend, da sie im Gegensatz zu vergleichbaren Pasten des oben beschriebenen Standes der Technik keine Thermoplaste umfasst. Überraschenderweise hat sich genau bei diesem Verhältnis von Baryt zu Halloysit gezeigt, dass die Paste in einem Röntgenbild sehr gut sichtbar ist und trotzdem die vorteilhaften Eigenschaften des Halloysit im medizinischen Einsatz zur Geltung kommen. Das Mineral Halloysit wirkt vorteilhaft blutstillend. Die Verwendung von den erfindungsgemäß nur relativ kleinen Mengen des Halloysit im Verhältnis zu Baryt erweist sich als kostengünstig, da der Halloysit ein sehr seltenes und schwer zu beschaffenes Mineral im Gegensatz zu Baryt ist. Das erfindungsgemäße Verhältnis ermöglicht zudem eine überraschend schnelle vorteilhafte gleichmäßige Durchmischung des Halloysit mit dem Baryt. Neben den genannten Vorteilen dieser erfindungsgemäßen Paste sind für die spätere dauerhafte Fixierung der Paste auf den textilen Gebilden und/oder anderweitigen röntgenkontrastunfähigen Erzeugnissen überraschend wenig weitere Komponenten von Bedarf. Diese Komponenten werden im weiteren Verlauf der Beschreibung im Näheren erläutert.

Dem Fachmann ist dabei ersichtlich, dass mit dem beschriebenen Volumenanteilen im Wesentlichen vier Volumenanteile Baryt zu im Wesentlichen einem Volumenteil Halloysit gemeint ist. Dementsprechend fallen auch die Verhältnisse wie beispielsweise 3,5; 3,6 ... 3,99 ...4,1 ... 4,999 Volumenanteile Baryt zu 0,5;0,6; ... 1,999 Volumenanteilen Halloysit unter die beschriebene Formulierung.

Begriffe wie im Wesentlichen, ungefähr, etwa, ca. etc. beschreiben bevorzugt einen Toleranzbereich von weniger als ± 40%, bevorzugt weniger als ± 20%, besonders bevorzugt weniger als ± 10 %, noch stärker bevorzugt weniger als ± 5% und insbesondere weniger als ± 1% und umfassen stets den exakten Wert. Ähnlich beschreibt bevorzugt Größen, die ungefähr gleich sind. Teilweise beschreibt bevorzugt zu mindestens 5 %, besonders bevorzugt zu mindestens 10 %, und insbesondere zu mindestens 20 %, in einigen Fällen zu mindestens 40 %.

Im Sinne der Erfindung ist eine Paste bevorzugt eine Salbe, eine Creme, ein Gel und/oder ein Feststoff-Flüssigkeitsgemisch (Suspension) mit einem hohen Gehalt an Festkörpern. Der Anteil an Festkörpern bezogen auf ein Gesamtvolumen oder einem Gesamtgewicht liegt bevorzugt bei ≥ 15 %. Die Viskosität der Paste liegt bei 20°C sowie bei Umgebungsdruck bevorzugt zwischen 5 *mPa* s und 2 × *10⁶ mPa* s, stärker bevorzugt zwischen *200 mPa* s und *3000 mPa s,* und besonders bevorzugt zwischen *500 mPa* s und *1500 mPa* s und insbesondere zwischen 550 *mPa* s und 650 *mPa* s. Der Vorteil einer Paste sind ihre guten Eigenschaften in Bezug auf ihre Fließfähigkeit und Weiterverarbeitung, sodass die erfindungsgemäße Paste für die Markierung von textilen Gebilden und/oder anderweitigen röntgenkontrastunfähigen Erzeugnissen besonders gut geeignet ist. Zudem ist die Herstellung einer solchen Paste ohne einen großen Aufwand (unter anderem Energieaufwand) möglich. Zusätzlich können alle Pasteneigenschaften mit kleinen Änderungen in der Zusammensetzung der Paste je nach Einsatzgebiet variiert bzw. angepasst werden.

In einer bevorzugten Ausführungsform sind textile Gebilde ausgewählt aus einer Gruppe umfassend Seile, Gewebe, Gewirke, Gestricke, Geflechte, Nähgewirke, Vliesstoffe und Filze. Dem Fachmann sind solche textilen Gebilde bekannt. Textile Gebilde sind flexible Erzeugnisse, welche viele Einsatzmöglichkeiten darbieten und besonders gut für die Verwendung als Wundauflage geeignet sind. Durch ihre biegeschlaffe Eigenschaft lassen sie sich besonders vorteilhaft flexibel an allen Körperteilen von Menschen und Tieren anlegen. Zudem gewährleisten Wundauflagen ein vorteilhaftes heilungsförderndes feuchtes Wundklima und sind in der Lage viel Wundflüssigkeiten wie Blut oder dergleichen aufzunehmen.

Im Sinne der Erfindung sind röntgenkontrastunfähig Erzeugnisse solche Materialien, die unter Einwirkung von Röntgenstrahlung in einem daraus entstehenden Röntgenbild nicht sichtbar sind. Die Erzeugnisse können beispielsweise bevorzugt aus Papier, Holz, Kunststoff etc. sein.

Baryt oder (BaSO₄) ist auch unter dem Namen Bariumsulfat bekannt. Baryt ist dem Fachmann bekannt und hat vorteilhafte Eigenschaften in Bezug auf die Absorption von Röntgenstrahlen. Überdies ist Baryt ein guter Füllstoff. Es erhöht die Oberflächenhärte von Kunststoffen. Zudem ist Baryt ein häufig vorkommendes Mineral, sodass es seine vorteilhaften Eigenschaften auch unter kostengünstige Aspekten in die erfindungsgemäße Paste einbringt.

Halloysit wird der Mineralklasse der Silikate zugeordnet. Innerhalb der Silikatminerale ist Halloysit ein Vertreter der Schichtsilikate (Phyllosilikate) und zudem der Gruppe der Tonminerale zugehörig. Halloysit ist das namensgebende Mineral der Halloysit-Gruppe, die aus den Mineralen Halloysit-7Å, Halloysit-10Ä und Hisingerit besteht. Halloysit-10Ä ist dem Fachmann auch unter dem Namen Endellit bekannt. Halloysite sind bipolar, d.h. die äußere Oberfläche der Tubes ist negativ geladen und die innere Oberfläche der Hohltubes ist positiv geladen. Der Halloysit liegt in Form von Nanotubes vor.

In einer bevorzugten Ausgestaltung der Erfindung ist das Halloysit ausgesucht aus Halloysit-7Å , Halloysit-10Ä und/oder von natürlichen Mischungen daraus. Dem Fachmann ist dabei bekannt, dass Å die Kurzbezeichnung für Angström und demnach eine physikalische Länge darstellt.

Es lag für den Fachmann nicht nahe, dass die bevorzugten Nanotubes eine überraschend gute blutstillende Wirkung haben, sodass die Paste neben der Röntgenkontrastfähigkeit auch unter wundheilenden Aspekten eine vorteilhafte Wirkung erzielt. Die Halloysit-Nanotubes sind weiterhin biokompatibel, und unterliegen nicht der EU-Nanopartikeldefinition (> 50 %; < 100 nm) und sind von der EPA als non toxic/ GRAS eingestuft.

In einer weiteren bevorzugten Ausführungsform weist die Paste Mineralstoffe umfassend Baryt (BaSO₄) und Halloysit in einem Verhältnis von:
- Bevorzugt 2 bis 6 Volumenteilen Baryt zu etwa einem Volumenteil Halloysit und;
- Stärker bevorzugt 3 bis 5 Volumenteilen Baryt zu etwa einem Volumenteil Halloysit und;
- insbesondere etwa 4 Volumenteilen Baryt zu etwa einem Volumenteil Halloysit.

Die beschriebenen Volumenverhältnisse von Baryt und Halloysit ermöglichen vorteilhaft die Sichtbarkeit auf einem Röntgenbild sowie ebenso eine positive Wirkung auf die Wundheilung. Es war überraschend, dass die beschriebenen Volumenanteile der Paste eine gute Temperaturstabilität verleihen.

In einer bevorzugten Ausführungsform weist die Paste bevorzugt einen höheren Volumenanteil an Baryt als Halloysit auf. So kann das Verhältnis bspw. auch wie folgt ausgestaltet sein (ohne darauf beschränkt zu sein):
- etwa 2 Volumenanteile Baryt zu etwa einem Volumenanteil Halloysit oder;
- etwa 3 Volumenanteile Baryt zu etwa 2 Volumenanteilen Halloysit oder;
- etwa 4 Volumenanteile Baryt zu etwa 3 Volumenanteilen Halloysit oder;
- etwa 5 Volumenanteile Baryt zu etwa 2 Volumenanteilen Halloysit.

Der hohe Volumenanteil an Baryt gewährleistet eine gute Sichtbarkeit bzw. Kontrastfähigkeit auf einem Röntgenbild, da insbesondere Baryt gute Absorptionseigenschaften aufweist. Zudem ist der Baryt ein oft vorkommendes Mineral, sodass die Beschaffung vorteilhaft kostengünstig erfolgt.

In einer bevorzugten Ausführungsform der Erfindung ist die Paste dadurch gekennzeichnet, dass die Paste einen Wasseranteil und einen Binderanteil umfasst. Vorteilhaft zeigt sich durch das Hinzufügen von Binder und Wasser eine verbesserte Konsistenz (Viskosität bei Umgebungsdruck und Umgebungstemperatur: 5 *mPa* s bis 2 × *10⁶ mPa* s) zur Weiterverarbeitung der Paste sowie eine vorteilhafte Bindung der einzelnen Pastenkomponenten. Der Wasseranteil sowie der Binderanteil sind variabel hinzufügbar, sodass die Viskosität und auch die Bindung der Pastenkomponenten je nach Anforderungen veränderbar sind. Eine besonders glatte Oberfläche eines zu markierenden Kunststoffes benötigt beispielsweise einen höheren Binderanteil in der Paste, als eine poröse Oberflächenstruktur eines zu markierenden Textils, um eine optimale Bindung zwischen der Paste und dem jeweils zu markierendem Objekt herstellen zu können.

Erfindungsgemäß ist ein Binder ein Stoff, der an Phasengrenzen anderer Stoffe chemische Bindungen herstellen und/oder begünstigt und/oder Effekte wie Kohäsion, Adsorption und Adhäsion bzw. Reibung auslösen und/oder vergrößert. Ein Binder verbindet Stoffe, indem er diese aufnimmt, anlagert, zusammenhält, vernetzt oder verklebt.

In einer bevorzugten Ausführungsform handelt es sich bei dem Wasser um demineralisiertes Wasser. Demineralisiertes Wasser weist eine sehr geringe elektrische Leitfähigkeit auf. Dies verhindert vorteilhaft die Agglomeration/ Verklumpung von Partikeln in der Paste
In einer weiteren bevorzugten Ausführungsform der Erfindung ist die Paste dadurch gekennzeichnet, dass die Paste mindestens ein Farbpigment umfasst. Der Vorteil einer solchen Paste ist die Sichtbarkeit für das menschliche Auge außerhalb einer Röntgentätigkeit. Durch einen starken Kontrast zwischen der Farbe der Paste und dem textilen Gebilde und/oder einem Erzeugnis, auf dem die Paste aufgebracht wird, kann ein Anwender deutlich sehen welche textilen Gebilde und/ oder Erzeugnisse markiert worden sind. Dabei kann jeder Farbe eine bestimmte Bedeutung zugeordnet werden. Die Paste kann zum Beispiel rot, blau, gelb, grün, orange, türkis, braun ausgestaltet sein. Die Farbe Blau könnte bei Wundauflagen dabei z.B. auf die Verwendung zur Behandlung chronischer Wunden hinweisen. Grün markierte Gepäcktaschen könnten in der Flugindustrie bspw. als schon kontrolliert und für ungefährlich erachtetes Gepäck erachtet werden.

Im Sinne der Erfindung sind Farbpigmente eine farbgebende Substanz. Im Gegensatz zu Farbstoffen bestehen sie aus Teilchen und sind im Anwendungsmedium praktisch unlöslich. Ein Anwendungsmedium ist der Stoff, in dem das Pigment eingearbeitet wird. Erfindungsgemäß ist das Anwendungsmedium eine Paste der eingangs genannten Art.

In einer bevorzugten Ausführungsform umfasst die Paste keine Farbpigmente, sodass sie nach Aufbringen auf dem textilen Gebilde und/oder röntgenkontrastunfähigem Erzeugnis für das menschliche Auge nicht sichtbar oder nur schwer erkennbar ist. Unter dem Einfluss von Röntgenstrahlen wird die Markierung auf einem Röntgenbild hingegen sichtbar. Somit kann die Markierung vorteilhaft als ein sogenanntes Wasserzeichen dienen. Wasserzeichen könnten zum Beispiel verwendet werden, um bei erster Inaugenscheinnahme nicht erkennbare Produktpiraterie, unter Röntgenstrahlen sichtbar zu machen. Ein Wasserzeichen könnte auch dafür dienen, um eigene Produkte zu markieren und Fälschungen zu erkennen.

In einer weiteren bevorzugten Ausführungsform der Erfindung ist die Paste nach einem der vorherigen Ansprüche dadurch gekennzeichnet, dass die Primärpartikelgröße von Baryt und/ oder Halloysit < 5 µm ist. Die Paste weist damit gute Eigenschaften in Bezug auf ihre Dichte und Viskosität auf. Insbesondere haben kleine Partikelgrößen den Vorteil, dass sie eine gute Durchmischung der Paste gewährleisten. Üblicherweise sinken Partikel (Feststoffe) durch ihre größere Dichte im Vergleich zur reinen Flüssigkeit langsam auf den Boden ab und bilden ein Sediment (Sedimentation). Es war für die Erfinder nicht vorherzusehen und daher überraschend, dass bei der beschrieben Partikelgröße von < 5 µm Sedimente im Wesentlichen vorteilhaft vermieden werden können.

In einer bevorzugten Ausführungsform der Erfindung ist vorgesehen, dass die Halloysit-Nanotubes die folgenden Abmessungen aufweisen: Innerer Durchmesser: 10 bis 20 Nanometer, äußerer Durchmesser: 50 bis 70 Nanometer und/oder Länge: 0,3 bis 4 Mikrometer. Es hat sich vorteilhaft gezeigt, dass diese Abmessungen in der Natur vorkommen und daher zum Gebrauch in der Paste nur mit üblichen Verfahren, wie Trocknung, Mahlung, Sichtung und Dispergierung weiterverarbeitet werden müssen.

Erfindungsgemäß sind Primärpartikel Partikel, die sich zu größeren Verbundsystemen (Agglomerate oder Aggregate) zusammenschließen können. Dabei weiß der Fachmann, dass die Partikelgrößen variieren können und es sich bei der Primärpartikelgröße bevorzugt um einen durchschnittlichen Wert handelt. In einer bevorzugten Ausgestaltung ist die Primärpartikelgröße von Baryt und Halloysit < 10 µm, stärker bevorzugt < 8 µm und insbesondere < 5 µm. Der Vorteil der beschriebenen Partikelgrößen ist die Möglichkeit einer guten und gleichmäßigen Durchmischung der Partikel in der Suspension (Paste).

In einer weiteren bevorzugten Ausführungsform der Erfindung ist die Paste dadurch gekennzeichnet, dass die Halloysitpartikel eine mittels Plasmatechnologie aufgebrachte Beschichtung mit atomarem Kupfer aufweisen. Vorteilhaft wirkt das aufgebrachte Kupfer überraschend antibakteriell. Die antibakterielle Wirkung ist vorteilhaft für die Behandlung von chronischen Wunden.

Dem Fachmann ist bekannt, dass unter der Beschichtung von Partikeln auch ein Coating zu verstehen ist. Unter Coating ist erfindungsgemäß das Beschichten oder Verkapseln von Partikeln mit Flüssigkeiten (Lösung, Suspension oder Schmelze) zu verstehen. Neben der Plasmatechnologie können ebenfalls Partikel durch ein Wirbelschicht-Coating Verfahren, ein Trommel-Coating Verfahren, Top-Spray-Coating Verfahren, Bottom-Spray-Coating Verfahren und einem Wurster-Verfahren beschichtet werden. Dem Fachmann sind diese Verfahren bekannt.

In einer bevorzugten Ausführungsform wird die vakuumbasierte Plasmabeschichtung unter Verwendung von Niederdruckplasmen für das Aufbringen einer Beschichtung auf die Halloysitpartikel appliziert, wobei insbesondere PVD-Technologie genutzt wird und dabei die Sputterdeposition in Schaukelkammer- oder Drehtrommelreaktoren Verwendung findet. Die Kupferpartikelgröße auf den Halloysitoberflächen beträgt 5 - 10 nm.

Dem Fachmann ist bekannt, dass atomares Kupfer insbesondere ein Synonym für ungeladenes (ladungsneutrales) Kupfer ist.

In einer weiteren bevorzugten Ausführungsform der Erfindung ist die Paste dadurch gekennzeichnet, dass der Binder ein Cellulose-Saccharid-Gemisch aufweist oder ein Acrylat ist. Diese Binder erweisen sich als besonders wirtschaftlich und weisen zudem besonders gute Bindungseigenschaften für die Komponenten der Paste auf. Acrylat weist überraschend schnelle aushärtende Eigenschaften auf, sodass dadurch vorteilhaft die Trocknungszeit kurz gehalten werden kann. Das Cellulose-Saccharid-Gemisch ist hingegen vorteilhaft sehr umweltfreundlich.

Bindemittel sorgen für den Zusammenhalt von Pulverpartikeln. Am häufigsten werden Bindemittellösungen wie Stärkeleister und Cellulosederivate eingesetzt. Polysaccharidlösungen mit niedriger Viskosität werden wegen der besseren zerfallsfördernden Eigenschaft (schnelles Lösen bzw. Zerfall in Wasser) bevorzugt. Bei feuchtigkeitsempfindlichen Wirkstoffen werden Bindemittel (Cellulosederivate wie z.B. Celluloseacetat, Methylcellulose, Ethylcellulose, Hydroxypropylmethylcellulose etc.) in organischen Lösungsmitteln eingesetzt. Mikrokristalline Cellulose kann auch in trockener Form Bindemitteleigenschaften ausüben, wodurch vorteilhaft kein Wasseranteil zur Bindung von Halloysit und Baryt notwendig ist.

In einer bevorzugten Ausführungsform der Erfindung ist das Mischungsverhältnis des Cellulose-Saccharid-Gemisch so ausgestaltet, dass sowohl Cellulose als auch Saccharid zu mindestens 0,01 Gew.-% bezogen auf ein Gesamtgewicht enthalten sind. Das Gemisch umfasst bevorzugt nur die beiden Stoffe (Cellulose und Saccharid). Das Gemisch hat vorteilhafterweise eine gute Bioverträglichkeit, bei überraschend guter Bindung.

In einer anderen bevorzugten Ausführungsform ist vorgesehen, dass das Bindemittel 90 % bis 95 % deionisiertes Wasser und 5 % bis 10 % Acrylat umfasst. Vorteilhafterweise erlaubt dies, die Paste besonders gut an das textile Gebilde und/oder Erzeugnis zu binden bzw. sie dort zu fixieren. Es war überraschend, dass hierfür keine weiteren Zusätze benötigt werden.

In einer weiteren bevorzugten Ausführungsform der Erfindung ist die Paste dadurch gekennzeichnet, dass die Paste einen Wasseranteil von ≥ 65 Gew.-%, einen Mineralstoffanteil von ≤ 30 Gew.-%, einen Binderanteil von ≤ 8 Gew.-% und einen Farbpigmentanteil von ≤ 0,02 Gew.-% umfasst. Insbesondere diese Komponentenmengen ergeben eine Paste, die besonders wirtschaftlich und qualitativ hochwertig die Sichtbarkeit unter dem Einfluss von Röntgenstrahlen gewährleistet, wobei zum Herstellen und Weiterverarbeiten der Paste vorteilhaft wenig Energie benötigt wird. Die beschriebene Menge von Farbpartikeln reicht überraschend gut aus, um der Paste eine gleichmäßige Farbgebung zu verleihen.

In einer weiteren bevorzugten Ausgestaltung umfasst die Paste folgende Mengenanteile: einen Wasseranteil von etwa 55 Gew.-% bis etwa 65 Gew.-%, einen Mineralstoffanteil von etwa 30 Gew.-% bis etwa 45 Gew.-%, einen Binderanteil von etwa 8 Gew.-% bis etwa 10 Gew.-% und einen Farbpigmentanteil von etwa ≤ 0,02 Gew.-% umfasst.

Ferner enthält die Erfindung eine Wundauflage, umfassend Verbundfasern und einer auf diese aufgebrachte Paste der eingangs genannten Art.

Im Sinne der Erfindung sind Wundauflagen im Allgemeinen als textile Gebilde zu sehen. Die Wundauflage gemäß der Erfindung kann bevorzugt auch als Verbandmittel eingesetzt werden. Hiermit bezeichnet man im Sinne der Erfindung jene Materialien, aus denen ein medizinischer Verband hergestellt wird. Sie werden deshalb auch als Verbandmaterial oder umgangssprachlich Verband(s)zeug bezeichnet. Sie werden oft in Form von Verbandkästen griffbereit gelagert. In weiterer Folge lässt sich zwischen sterilem oder zumindest keimarmen Wundauflagen und nicht sterilem Befestigungsmaterial unterscheiden. Ebenfalls bevorzugt sind auch gebrauchsfertige, kombinierte Zusammenstellungen von Wundauflage und Befestigungsmaterial. Sterile Wundauflagen sind Kompressen und Verbandstücher. Zum Befestigungsmaterial gehören bevorzugt Mullbinden, Idealbinden, elastische Fixierbinden, Trikotschlauchbinden und Dreiecktücher.

Wunden und Wundverletzungen werden nach Art der Einwirkung und ihrem Aussehen nach in verschiedene Wundarten unterteilt und unterschiedlich behandelt. Eine Schnittwunde entsteht zum Beispiel durch eine Schnittverletzung und ist in der Regel glattrandig. Meist bluten Schnittwunden sehr stark. Sie gehören zu den häufigsten Verletzungen im Alltag. Neben Schnittwunden gibt es auch Risswunden, Stichwunden, Bisswunden, Schusswunden, Quetschwunden, Schürfwunden, Platzwunden, Kratzwunden, Verbrennungen und Verätzungen.

Der Vorteil einer Wundauflage, welche mit der oben beschriebenen Paste markiert wird, ist die Möglichkeit in Röntgenaufnahmen die Wundauflage zu lokalisieren. Die bevorzugte Wundauflage stillt Blutungen in der Chirurgie, bei der Ersten Hilfe, bei der Notfallversorgung oder bei der Versorgung chronischer Wunden besonders effizient. Es lag für den Fachmann nicht nahe, dass die Wundauflage gemäß der bevorzugten Ausführungsform zu einer verbesserten Versorgung einer akuten oder chronischen Wunde führt, da er nicht davon ausgehen konnte, dass eine geringe Menge Halloysitpartikel schon eine solche effektive Wirkung erzielen konnte. Es war überraschend, dass die erfindungsgemäße Wundauflage ebenfalls Wundhämatome und -serome minimiert. Es war zudem für die Erfinder nicht vorherzusehen, dass es vorteilhaft zu einem geringeren Auseinanderweichen von Wundrändern durch Einsatz der Wundauflage der genannten Art kommt.

In einer bevorzugten Ausführungsform werden Wundauflagen auf äußere Wunden gelegt, um das Eindringen von Fremdkörpern in die Wunde zu verhindern und Blut sowie Wundexsudat aufzunehmen. Zudem können Wundauflagen ein heilungsförderndes feuchtwarmes Wundklima gewährleisten, durch enthaltene Substanzen Schmerzen mindern, die Wundheilung fördern oder antimikrobiell wirksam werden.

In einer weiteren Ausgestaltung werden Wundauflagen zur inkorporalen Applikation verwendet. Insbesondere in diesem Fall ist die Bedeutung einer Sichtbarkeit in Röntgenbilder von hoher Relevanz.

In einer weiteren bevorzugten Ausführungsform ist die Wundauflage dadurch gekennzeichnet, dass die Paste 80 Volumenprozent Baryt und 20 Volumenprozent Halloysit in Bezug auf das Gesamtvolumen der Mineralstoffe umfasst.

In einer weiteren bevorzugten Ausführungsform weist die Paste
a) Bevorzugt 60 - 95 Volumenprozent Baryt und 5 - 40 Volumenprozent Halloysit;
b) besonders bevorzugt 70 - 85 Volumenprozent Baryt und 15 - 30 Volumenprozent Halloysit und;
c) insbesondere 80 Volumenprozent Baryt und 20 Volumenprozent Halloysit.

in Bezug auf das Gesamtvolumen der Mineralstoffe auf.

Überraschenderweise hat sich genau bei der Volumenverteilung von Baryt und Halloysit gezeigt, dass die Markierung der Wundauflage in einem Röntgenbild sehr gut sichtbar ist und trotzdem die vorteilhaften Eigenschaften des Halloysit zur Geltung kommen.

In einer anderen bevorzugten Ausgestaltung können ebenfalls weitere Volumenanteilvarianten umfasst sein. Erfindungsgemäß gilt stets, dass Baryt einen größeren Volumenanteil als Halloysit in Bezug auf ein Gesamtvolumen der Mineralstoffe in der Paste aufweist. Bevorzugte Anteile sind weiter oben beschrieben. Der große Anteil an Baryt ist vorteilhaft für die Sichtbarkeit in einem Röntgenbild, da die Absorptionsrate von Röntgenstrahlen vorteilhaft groß ist.

In einer weiteren bevorzugten Ausführungsform ist das Verfahren zur Aufbringung einer Paste auf einem textilen Gebilde und/oder anderweitigen Erzeugnissen dadurch gekennzeichnet, dass die Paste mittels eines Druckverfahrens auf das textile Gebilde und/oder auf das anderweitige Erzeugnis aufgebracht wird. Durch ein Druckverfahren können akkurate, detailreiche Muster sowie eine große Menge von textilen Gebilden und/oder Erzeugnissen in hoher Geschwindigkeit bedruckt werden. Druckverfahren ermöglichen im Gegensatz zu bekannten Sprüh- und Tauchtechnologien des Standes der Technik eine exakte und variierende Imprägnation der textilen Gewebefläche, sodass bevorzugt vollständig auf die Zugabe weiterer Chemikalien und Agentien, wie dies im Stand der Technik vorgesehen ist, verzichtet werden kann. Es war überraschend, dass die aufgebrachten Partikel der Paste sich vorteilhaft nicht aus der Wundauflage herauslösen.

In einer bevorzugten Ausgestaltung ist das Druckverfahren ausgewählt aus der Gruppe von Verfahren umfassend: Hochdruck, Tiefdruck, Durchdruck und/oder Flachdruck. Dem Fachmann sind alle Verfahren bekannt.

In einer bevorzugten Ausführungsform ist das Verfahren dadurch gekennzeichnet, dass das Druckverfahren ein Siebdruckverfahren und/oder ein Rotationsdruckverfahren ist. Beide Verfahren sind aus dem Stand der Technik bekannt. Die Paste der eingangs genannten Art lässt sich besonders gut mit derartigen Druckverfahren auf ein textiles Gewebe und/oder anderweitiges röntgenkontrastunfähiges Erzeugnis auftragen. Der Vorteil des Siebdruckverfahrens besteht darin, dass durch verschiedene Gewebefeinheiten der Pastenauftrag variiert werden kann, so dass hohe Pastenschichtdicken für die Markierung erreicht werden können. Zudem können mit dem Siebdruckverfahren ebenfalls nichtflexible starre Gegenstände bedruckt werden. Rotationsdruckverfahren ermöglichen vorteilhaft eine besonders schnelle Bedruckung von textilen Gebilden.

In einer bevorzugten Ausführungsform ist das Verfahren dadurch gekennzeichnet, dass verschiedene Druckmotive ausgewählt werden können. Vorteilhaft können Druckmotive eine bestimmte Bedeutung aufweisen, sodass das dem Nutzer durch einen kurzen Blick Informationen über das bedruckte textile Gebilde und/oder röntgenkotrastunfähige Erzeugnis bereitgestellt werden. Solche Informationen könnten beispielsweise vor einer Gefahr warnen, einen Adressaten aufweisen oder das Ergebnis einer zu einem früheren Zeitpunkt durchgeführten Überprüfung aufzeigen.

In einer bevorzugten Ausführungsform der Erfindung sind die Druckmotive als Streifen und/oder Wellen ausgestaltet. Sie können durch die oben benannten Druckverfahren jedoch auch detaillierte Muster oder Bilder umfassen. Zum Beispiel: ein Kreuz, ein Dreieck, eine Abbildung des markierten Gegenstandes, ein Piktogramm und viele mehr.

In einer bevorzugten Ausführungsform ist das Verfahren dadurch gekennzeichnet, dass nach dem Aufdrucken der Paste eine temperaturgeführte Trocknung durchgeführt wird. Im Sinne der Erfindung umfasst die temperaturgeführte Trocknung eine kontrollierte Trocknung. Das heißt die Paste wird nach dem Aufdrucken an einer definierten Temperatur getrocknet. Bevorzugt ist die Temperatur gleichmäßig im Trocknungsraum überall gleich hoch. Diese Temperatur ist bevorzugt die Trocknungstemperatur. Der Vorteil einer solchen kontrollierten Trocknung ist die Prozessüberwachung, die dadurch ermöglicht wird. Überdies wird dadurch ein geregelter Prozess erst möglich, der für jeden Druck wiederholbar ist. Die Prozessüberwachung steigert vorteilhaft die Qualität der Produkte, sodass die Paste eine erhöhte Beständigkeit (Haftung auf den zu bedruckenden Erzeugnissen) aufweist.

In einer bevorzugten Ausführungsform ist das Verfahren dadurch gekennzeichnet, dass die Trocknung bei einer Trocknungstemperatur von <100 °C ausgeführt wird. Die vergleichsweise (im Vergleich zu den eingangs beschriebenen Verfahren des Standes der Technik) tiefe Temperatur zur Trocknung der aufgebrachten Paste benötigt vorteilhaft wenig Energie und ist trotzdem ausreichend für eine Haftung der Paste auf dem textilen Gebilde und/oder Erzeugnis.

In einer weiteren bevorzugten Ausgestaltung der Erfindung kann die Trocknungstemperatur aus folgenden Temperaturbereichen ausgewählt sein: <98 °C, <96°C, < 95°C oder <90°C. Auch wenn tiefe Temperaturen eine längere Trocknungszeit hervorrufen, ist der gesamte Energieaufwand überraschenderweise trotzdem geringer. Die beschriebenen Trocknungstemperaturen ermöglichen eine vorteilhafte gleichmäßige Trocknung der Paste, wodurch sich die Beständigkeit erhöht.

In einem letzten Aspekt weist die Erfindung eine Verwendung der oben genannten Wundauflage für die Versorgung von Wundverletzungen auf.

In einer bevorzugten Ausführungsform der Verwendung der erfindungsmäßen Wundauflage werden stark blutenden Wunden zunächst gereinigt, anschließend wird bevorzugt über der sterilen erfindungsgemäßen Wundauflage zur Vermeidung eines größeren Blutverlusts ein Druckverband angelegt.

## Patentansprüche

1. Paste für die Markierung von Wundauflagen
**dadurch gekennzeichnet, dass**
die Paste Mineralstoffe, umfassend Baryt (BaSO₄) und Halloysit in einem Verhältnis von vier Volumenteilen Baryt zu einem Volumenteil Halloysit aufweist, und
die Primärpartikelgröße von Baryt und/oder Halloysit < 5 µm ist.

2. Paste nach Anspruch 1
**dadurch gekennzeichnet, dass**
die Paste einen Wasseranteil und einen Binderanteil umfasst.

3. Paste nach einem der vorherigen Ansprüche
**dadurch gekennzeichnet, dass**
die Paste mindestens ein Farbpigment umfasst.

4. Paste nach einem der vorherigen Ansprüche
**dadurch gekennzeichnet, dass**
die Halloysitpartikel eine mittels Plasmatechnologie aufgebrachte Beschichtung mit atomarem Kupfer aufweisen.

5. Paste nach einem der vorherigen Ansprüche
**dadurch gekennzeichnet, dass**
der Binder ein Cellulose-Sacharid-Gemisch aufweist oder ein Acrylat ist.

6. Paste nach einem der vorherigen Ansprüche
**dadurch gekennzeichnet, dass**
die Paste einen Wasseranteil von ≥ 65 Gew.-%, einen Mineralstoffanteil von ≤ 30 Gew.-%, einen Binderanteil von ≤ 8 Gew.-% und einen Farbpigmentanteil von ≤ 0,02 Gew.-% umfasst.

7. Zusammensetzung umfassend Halloysit und Baryt in einem Verhältnis von vier Volumenteilen Baryt zu einem Volumenteil Halloysit zur Anwendung in einem Verfahren für die Versorgung von Wundverletzungen, wobei die Zusammensetzung in Form einer Paste gemäß einem der vorhergehenden Ansprüche vorliegt und auf einer Wundauflage aufgetragen ist.

8. Wundauflage umfassend Verbundfasern und einer auf diese aufgebrachte Paste nach einem der Ansprüche 1 bis 6.

9. Wundauflage nach Anspruch 8
**dadurch gekennzeichnet, dass**
die Paste 80 Volumenprozent Baryt und 20 Volumenprozent Halloysit in Bezug auf das Gesamtvolumen der Mineralstoffe umfasst.

10. Verfahren zur Aufbringung einer Paste gemäß der Ansprüche 1-6 auf einer Wundauflage **dadurch gekennzeichnet, dass**
die Paste mittels eines Druckverfahrens auf die Wundauflage und/oder auf das anderweitige Erzeugnis aufgebracht wird, und
die Trocknung bei einer Trocknungstemperatur von <100 °C ausgeführt wird.

11. Verfahren nach Anspruch 10
**dadurch gekennzeichnet, dass**
das Druckverfahren ein Siebdruckverfahren und/oder ein Rotationsdruckverfahren ist.

12. Verfahren nach den Ansprüchen 10 oder 11
**dadurch gekennzeichnet, dass**
verschiedene Druckmotive ausgewählt werden können.

13. Verfahren nach den Ansprüchen 10 - 12
**dadurch gekennzeichnet, dass**
nach dem Aufdrucken der Paste eine temperaturgeführte Trocknung durchgeführt wird.

14. Verfahren nach den Ansprüchen 10 - 13
**dadurch gekennzeichnet, dass**
die Trocknung bei einer Trocknungstemperatur von < 90 °C ausgeführt wird.

15. Wundauflage nach Anspruch 8 oder 9, wobei die Wundauflage für die Versorgung von Wundverletzungen konfiguriert ist.

## Claims

1. A paste for marking wound dressings,
**characterized in that**
the paste includes minerals comprising barite (BaSO₄) and halloysite in a ratio of four parts by volume of barite to one part by volume of halloysite; and
the primary particle size of barite and/or halloysite is < 5 µm.

2. The paste according to claim 1,
**characterized in that**
the paste comprises a water content and a binder content.

3. The paste according to any one of the preceding claims,
**characterized in that**
the paste comprises at least one color pigment.

4. The paste according to any one of the preceding claims,
**characterized in that**
the halloysite particles include a coating with atomic copper applied by plasma technology.

5. The paste according to any one of the preceding claims,
**characterized in that**
the binder includes a cellulose/saccharide mixture or is an acrylate.

6. The paste according to any one of the preceding claims,
**characterized in that**
the paste comprises a water content of ≥ 65 % by weight, a mineral content of ≤ 30 % by weight, a binder content of ≤ 8 % by weight and a color pigment content of ≤ 0.02 % by weight.

7. A composition, comprising halloysite and barite in a ratio of four parts by volume of barite to one part by volume of halloysite for use in a method of treating wound injuries, the composition being in the form of a paste according to any one of the preceding claims and being applied to a wound dressing.

8. A wound dressing, comprising composite fibers and a paste as claimed in any one of claims 1 to 6 applied thereto.

9. The wound dressing according to claim 8,
**characterized in that**
the paste comprises 80 % by volume of barite and 20 % by volume of halloysite based on the total volume of minerals.

10. A method of applying a paste according to claims 1-6 to a wound dressing,
**characterized in that**
the paste is applied by a printing method to the wound dressing and/or to the
other product; and
drying is carried out at a drying temperature of <100 °C.

11. The method according to claim 10,
**characterized in that**
the printing method is a screen printing method and/or a rotary printing method.

12. The method according to claims 10 or 11,
**characterized in that**
different print motifs can be selected.

13. The method according to claims 10-12,
**characterized in that,**
after the paste has been printed on, temperature-controlled drying is carried out.

14. The method according to claims 10-13,
**characterized in that**
drying is carried out at a drying temperature of <90 °C.

15. The wound dressing according to claim 8 or 9, wherein the wound dressing is configured for treating wound injuries.

## Revendications

1. Pâte pour le marquage de pansements
**caractérisée en ce que**
la pâte contient des minéraux comprenant de la baryte (BaSO₄) et de l'halloysite dans un rapport de quatre parties en volume de baryte à une partie en volume d'halloysite, et
la taille des particules primaires de baryte et/ou d'halloysite est < 5 pm.

2. Pâte selon la revendication 1
**caractérisée en ce que**
la pâte contient une partie eau et une partie liant.

3. Pâte selon l'une des revendications précédentes
**caractérisée en ce que**
la pâte contient au moins un pigment coloré.

4. Pâte selon l'une des revendications précédentes
**caractérisée en ce que**
les particules d'halloysite sont revêtues d'un revêtement avec du cuivre atomique appliqué au moyen de technologie plasma.

5. Pâte selon l'une des revendications précédentes
**caractérisée en ce que**
le liant est un mélange de cellulose et de saccharide ou un acrylate.

6. Pâte selon l'une des revendications précédentes
**caractérisée en ce que**
la pâte contient une teneur en eau ≥ 65 % en poids, une teneur en minéraux ≤ 30 % en poids, une teneur en liant ≤ 8 % en poids et une teneur en pigment coloré ≤ 0,02 % en poids.

7. Composition comprenant de l'halloysite et de la baryte dans un rapport de quatre parties en volume de baryte à une partie en volume d'halloysite pour une utilisation dans un procédé de traitement de lésions, dans laquelle la composition est sous la forme d'une pâte selon l'une des revendications précédentes et est appliquée sur un pansement.

8. Pansement comprenant des fibres composites et une pâte appliquée sur celles-ci selon l'une des revendications 1 à 6.

9. Pansement selon la revendication 8
**caractérisé en ce que**
la pâte contient 80 % en volume de baryte et 20 % en volume d'halloysite par rapport au volume total des minéraux.

10. Procédé d'application d'une pâte selon les revendications 1 à 6 sur un pansement
**caractérisé en ce que**
la pâte est appliquée au moyen d'un procédé d'impression sur le pansement et/ou sur
un autre produit, et
le séchage est effectué à une température de séchage < 100 °C.

11. Procédé selon la revendication 10
**caractérisé en ce que**
le procédé d'impression est un procédé d'impression sérigraphique et/ou un procédé d'impression rotative.

12. Procédé selon les revendications 10 ou 11
**caractérisé en ce que**
différents motifs d'impression peuvent être sélectionnés.

13. Procédé selon les revendications 10 à 12
**caractérisé en ce qu'un**
séchage à température contrôlée est effectué après l'impression de la pâte.

14. Procédé selon les revendications 10 à 13
**caractérisé en ce que**
le séchage est effectué à une température de séchage < 90 °C.

15. Pansement selon la revendication 8 ou 9, dans lequel le pansement est configuré pour soigner des blessures.
